(19) Europäisches Patentamt
European Patent Office
Office européen des brevets

(11) Publication number: **0 345 842 A2**

(12) **EUROPEAN PATENT APPLICATION**

(21) Application number: 89201226.1

(51) Int. Cl.4: **C11D 1/645 , A61K 7/08**

(22) Date of filing: 17.05.89

(30) Priority: 27.05.88 US 199542
01.05.89 US 344292
27.04.89 US 342431

(43) Date of publication of application:
13.12.89 Bulletin 89/50

(84) Designated Contracting States:
AT BE CH DE FR GB GR IT LI LU NL SE

(71) Applicant: THE PROCTER & GAMBLE COMPANY
One Procter & Gamble Plaza
Cincinnati Ohio 45202(US)

(72) Inventor: Walley, Darlene Rose
9576 Carriage Run Circle
Loveland Ohio 45140(UA)

(74) Representative: Canonici, Jean-Jacques et al
Procter & Gamble European Technical
Center N.V. Temselaan 100
B-1820 Strombeek-Bever(BE)

(54) **Fabric softening compositions containing mixtures of substituted imidazoline esters and quartenized ester-ammonium salts.**

(57) Shelf-stable biodegradable fabric softening compositions are provided comprising mixtures of a substituted imidazoline ester, a quaternized ester-ammonium salt, and a liquid carrier. These biodegradable compositions have improved product stability, dispersibility, and concentratability, as well as excellent fabric softening characteristics.

EP 0 345 842 A2

# FABRIC SOFTENING COMPOSITIONS CONTAINING MIXTURES OF SUBSTITUTED IMIDAZOLINE ESTERS AND QUATERNIZED ESTER-AMMONIUM SALTS

## TECHNICAL FIELD

The present invention relates to textile treatment compositions. In particular, it relates to textile treatment compositions for use in the rinse cycle of a textile laundering operation to provide fabric softening. static control benefits, the compositions being characterized by excellent storage stability, concentratability characteristics and softening performance, as well as by biodegradability. The compositions herein can also be used to treat fabrics in hot air clothes dryers and can be incorporated into through-the-wash detergent compositions, as well as into hair conditioner compositions.

## BACKGROUND OF THE INVENTION

Textile treatment compositions suitable for providing fabric softening and static control benefits during laundering are well-known in the art and have found wide-scale commercial application. Conventionally, rinse-added fabric softening compositions contain, as the active softening component, substantially water-insoluble cationic materials having two long alkyl chains. Typical of such materials are ditallow dimethyl ammonium chloride and imidazolinium compounds substituted with two stearyl groups. These materials are normally prepared in the form of a dispersion in water. It is generally not possible to prepare such aqueous dispersions with more than about 10% of cationic materials without encountering intractable problems of product viscosity and stability, especially after storage at elevated temperatures. Such high concentration compositions are frequently unpourable and may have inadequate dispensing and dissolving characteristics in rinse water. This physical restriction on softener concentration naturally limits the level of softening performance achievable without using excessive amounts of product, and also adds substantially to the costs of distribution and packaging.

Accordingly, it would be highly desirable to prepare physically-acceptable textile treatment compositions containing relatively high levels of water-insoluble cationic softener materials and exhibiting excellent softening performance.

It would also be desirable to have fabric softening compositions which are storage-stable and also which are biodegradable. However, materials which may be biodegradable are often difficult to formulate as stable liquid compositions.

It is an object of this invention to provide a storage-stable, biodegradable fabric softening composition. It is a further object to provide such materials in the form of liquid products, including concentrates, suitable for use in the rinse cycle of a textile laundering operation, and in sheet form for use in clothes dryers. These and other objects are obtained using the present invention, as will be seen from the following disclosure.

Cationic softener materials are normally supplied by the manufacturer in the form of a slurry containing about 70%-80% of active material in an organic liquid such as isopropanol, sometimes containing a minor amount of water (up to about 10%). Retail fabric softening compositions are then prepared by dispersion of the softener slurry in warm water under carefully controlled conditions. The physical form and dispersibility constraints of these industrial concentrates, however, are such as to preclude their direct use by the domestic consumer; indeed, they can pose severe processing problems even for the industrial supplier of retail fabric softening compositions.

The use of various quaternized ester-ammonium salts as cationic fabric softening agents is known in the art. See, for example, U.S.

Patent 4,339,391, Hoffmann et al., issued July 13, 1982, for a series of quaternized ester-ammonium salts which function as fabric softeners. Various quaternized ester-ammonium salts are commercially available under the tradenames SYNPROLAM FS from ICI and REWOQUAT from REWO. Similarly, methods for preparing various quaternized ester-amine compounds are known in the art. See, for example, U.S. Patent 3,342,840, Sobolev, issued September 19, 1967, U.S. Patent 3,872,138, Ogatu, issued March 18, 1975, and Japanese Laid Open Publication 49-1510, assigned to Gosei Chem. Ind. Co., published January 9, 1974.

Unfortunately, although quaternized ester-ammonium salts are believed to be rapidly biodegradable,

they are more subject to hydrolysis than are conventional cationic softening agents (e.g., ditallow dimethyl ammonium chloride and analogs thereof) and hence can encounter phase and chemical stability problems upon prolonged shelf storage. The product stability and viscosity problems become increasingly more unmanageable in concentrated aqueous dispersions.

Another class of nitrogenous materials that is sometimes used as the active component in rinse-added fabric softening compositions comprises the nonquaternary amide-amines and ester-amines. A commonly used material is the reaction product of higher fatty acids with a polyamine selected from the group consisting of hydroxyalkylenediamines and dialkylenetriamines and mixtures thereof. An example of these materials is the reaction product of higher fatty acids and hydroxyethylethylenediamine (see "Condensation Products from $\beta$-Hydroxyethylethylenediamine and Fatty Acids or Their Alkyl Esters and Their Application as Textile Softeners in Washing Agents," H. W. Eckert, Fette-Seifen-Anstrichmittel, September 1972, pages 527-533). These materials, along with other cationic quaternary ammonium salts and imidazolinium salts, are taught to be softening actives in fabric softening compositions. (See for example, U.S. Patents 4,460,485, Rapisarda et al., issued July 17, 1984; 4,421,792, Rudy et al., issued December 20, 1983; and 4,327,133, Rudy et al., issued April 27, 1982.)

The use of various imidazoline derivatives as fabric conditioning agents is known. For example, British Patent Specification 1,565,808, published April 23, 1980, discloses textile fabric softener compositions consisting of an aqueous dispersion of imidazoline ester derivatives. Similarly, methods for preparing various imidazoline derivatives are known in the art. See for example, U.S. Patent 4,233,451, Pracht, issued November 11, 1980, U.S. Patent 4,189,593, Wechsler et al., issued February 19, 1980, and Japanese Laid Open Publication 61-291571.

U.S. Patent 4,661,269, Trinh et al., issued April 28, 1987, discloses fabric softening compositions containing the reaction products of higher fatty acids with a polyamine selected from the group consisting of hydroxyalkylalkylenediamines, dialkylenetriamines, and mixtures thereof, and cationic nitrogenous salts having only one long chain acyclic aliphatic $C_{15}$-$C_{22}$ hydrocarbon group.

Various solutions to the problem of preparing concentrated fabric softening compositions suitable for consumer use have been addressed in the art. See, for example, U.S. Patents 4,426,299, issued January 17, 1984, and 4,401,578, issued August 30, 1983, Verbruggen, which relate to paraffin, fatty acids and ester extenders in softener concentrates as viscosity control agents.

European Patent 0,018,039, Clint et al., issued March 7, 1984, relates to hydrocarbons plus soluble cationic or nonionic surfactants in softener concentrates to improve viscosity and stability characteristics.

U.S. Patent, 4,454,049, MacGilp et al., issued June 12, 1984, discloses concentrated liquid textile treatment compositions in the form of isotropic solutions comprising water-insoluble di-$C_{16}$-$C_{24}$ optionally hydroxy-substituted alkyl, alkaryl or alkenyl cationic fabric softeners, at least about 70% of the fabric softener consisting of one or more components together having a melting completion temperature of less than about 20°C, a water-insoluble nonionic extender, especially $C_{10}$-$C_{40}$ hydrocarbons or esters of mono- or polyhydric alcohols with $C_8$-$C_{24}$ fatty acids, and a water-miscible organic solvent. The concentrates have improved formulation stability and dispersibility, combined with excellent fabric softening characteristics.

U.S. Patent 4,439,330, Ooms, issued March 27, 1984, teaches concentrated fabric softeners comprising ethoxylated amines.

U.S. Patent 4,476,031, Ooms, issued October 9, 1984, teaches ethoxylated amines or protonated derivatives thereof, in combination with ammonium, imidazolinium, and like materials.

The use of alkoxylated amines, as a class, in softener compositions is known (see, for example, German Patent Applications 2,829,022, Jakobi and Schmadel, published January 10, 1980, and 1,619,043, Mueller et al., published October 30, 1969, and U.S. Patents 4,076,632, Davis, issued February 28, 1978, and 4,157,307, Jaeger et al., issued June 5, 1979).

U.S. Patent 4,422,949, Ooms, issued December 27, 1983, relates to softener concentrates based on ditallow dimethyl ammonium chloride (DTDMAC), glycerol monostearate and polycationics.

In United Kingdom Application, 2,007,734A, Sherman et al., published May 23, 1979, fabric softener concentrates are disclosed which contain a mixture of fatty quaternary ammonium salts having at least one $C_8$-$C_{30}$ alkyl substituent and an oil or substantially water-insoluble compound having oily/fatty properties. The concentrates are said to be easily dispersed/emulsified in cold water to form fabric softening compositions.

Concentrated dispersions of softener material can be prepared as described in European Patent Application 406 and United Kingdom Patent Specification 1,601,360, Goffinet, published October 28, 1981, by incorporating certain nonionic adjunct softening materials therein.

As can be seen, the various solutions to the specific problem of preparing fabric softening compositions in concentrated form suitable for consumer use have not been entirely satisfactory. It is generally known (for

example, in U.S. Patent No. 3,681,241, Rudy, issued August 1, 1972) that the presence of ionizable salts in softener compositions does help reduce viscosity. This approach, however, is ineffective in compositions containing more than about 12% of dispersed softener, inasmuch as the level of ionizable salts necessary to reduce viscosity to any substantial degree has a seriously detrimental effect on product stability.

It has now been discovered that fabric softener compositions containing mixtures of specific substituted imidazoline esters and quaternized ester-ammonium salts exhibit surprisingly high levels of fabric softening activity relative to their individual components. These compositions additionally exhibit the benefit of high concentratability of softener active while retaining the high levels of fabric softening activity. Moreover, neither the use of a mixture of these fabric softening compounds in laundry fabric softening compositions, nor the desirable fabric softener/viscosity/stability/biodegradability properties of fabric softening compositions containing mixtures of these compounds, including concentrates, appears to have been appreciated heretofore.

## SUMMARY OF THE INVENTION

According to the present invention a shelf-stable/biodegradable, fabric softening composition is provided in the form of an aqueous dispersion comprising:

(a) from about 1% to about 20% by weight of a quaternized ester-ammonium softening compound selected from the group consisting of:

(i) 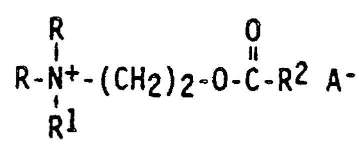

(ii) 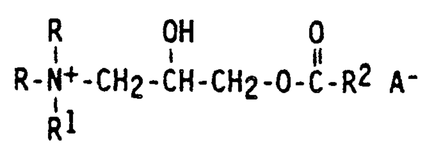

and mixtures thereof, wherein each R substituent is independently selected from $C_1$-$C_6$ alkyl, alkenyl or hydroxyalkyl groups, $R^1$ is a $C_{14}$-$C_{22}$ hydrocarbyl group, $R^2$ is a $C_{13}$-$C_{21}$ hydrocarbyl group, and $A^-$ is a softener compatible anion;

(b) from about 1% to about 35% by weight of a substituted imidazoline ester softening compound having the formula

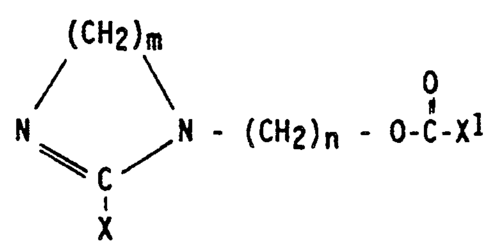

wherein X and $X^1$ are, independently, $C_{11}$-$C_{21}$ hydrocarbyl groups, and m and n are, independently, from about 2 to about 4; provided that the total concentration of component (a) and component (b) softening compounds does not exceed about 40% by weight; and

(c) from about 60% to about 98% of a liquid carrier.

While not intending to be limited by theory, it is believed that the ester moiety lends biodegradability to these softening compounds (i.e., both the quaternized ester-ammonium salts and the substituted imidazoline esters) whereas the fact that in each compound only a single ester group is present provides sufficient hydrolytic stability that mixtures of the compounds can be stably formulated as liquid compositions, under the conditions disclosed hereinafter.

Compositions containing only the quaternary ester-ammonium compounds will typically exhibit high levels of softening performance relative to compositions containing only the imidazoline ester compounds. On the other hand, compositions containing only the imidazoline ester compounds can be formulated at higher softener active concentrations than compositions containing only quaternized ester-ammonium compounds. However, it has been discovered that compositions comprising a mixture of quaternized ester-ammonium compounds and imidazoline ester compounds can exhibit improved softening performance relative to that which would be expected from such mixture by one of ordinary skill in the art. Furthermore, such compositions can be prepared at high levels of softener concentration while retaining surprisingly high levels of softener performance.

Additionally, the compositions of the present invention exhibit rapid biodegradability relative to compositions containing conventional fabric softening agents such as ditallow dimethyl ammonium chloride (DTDMAC) and ditallow imidazoline.

Because the quaternized ester-ammonium compounds present in the mixture are cationic, the compositions provide not only fiber and fabric softness benefits, they also provide anti-static benefits.

The liquid compositions of the present invention are preferably formulated at a pH of from about 2.0 to about 5.0, most preferably at a pH of from about 2.0 to about 3.5, to provide good storage stability.

The preferred liquid compositions herein have the softening compounds present as particles dispersed in the liquid carrier. The particles are preferably sub-micron size, generally having average diameters in the range of about 0.10-0.50 micron. Such particle dispersions can optionally be stabilized against settling by means of standard non-basic emulsifiers, especially nonionic extenders.

Importantly, the liquid compositions herein are substantially is free (generally, less than about 1% by weight) of free (i.e., unprotonated) amines, since free amines can catalyze decomposition of the ester-ammonium softening compounds on storage. If minor amounts of amines are present, they should be protonated with acid during the formulation of the compositions. Strong acids, such as $H_3PO_4$ and HCl, can be used for this purpose.

The low viscosities exhibited by dispersions of particles of the softening compounds described herein allow them to be formulated as water-dilutable fabric softener "high concentrates" which contain from about 15% to about 35% by weight of fabric softener active. Such high concentrates may be conveniently packaged in pouches, which can be diluted with water by the user to produce "single-strength" softeners (typically, 3-8% concentration of softener active).

The compounds herein can also be formulated as solids,.for example, in combination with particulate carriers as particulate fabric softening and antistatic compositions. Solid compositions can contain from about 1% to about 40% of softening compound (a) and from about 1% to about 40% softening compound (b), with the weight ratio of softening compound (a) to softening compound (b) preferably in the range of from about 0.04:1 to about 1.5:1. When formulated as solids, the pH and presence or absence of amines are, of course, not as critical as with the liquid compositions, since stability to hydrolysis on storage is not so problematic.

Other solid compositions herein have the compounds releasably affixed to sheet materials to provide fabric softening and antistatic compositions in sheet form which can be used in hot air clothes dryers.

The invention also encompasses a method of softening fibers (including hair) or fabrics, or imparting an antistatic finish thereto, comprising contacting said fibers or fabrics with a composition of the above-disclosed type.

All percentages, ratios and proportions herein are by weight, unless otherwise specified.

## DETAILED DESCRIPTION OF THE INVENTION

The compositions of the present invention are based upon the discovery that aqueous fabric softening compositions containing a mixture of (a) quaternized ester-ammonium softening compounds and (b) substituted imidazoline ester softening compounds exhibit improved softening performance relative to that which would be expected from such mixture by one of ordinary skill in the art. Furthermore, such compositions can be prepared at high levels of softener concentration while retaining surprisingly high levels of softener performance. These compositions are characterized by excellent storage stability, concentratability, and viscosity characteristics, as well as by high biodegradability. The compositions comprise a mixture of the two types of fabric softening compounds (i.e., groups (a) and (b) above) and a liquid carrier. The weight ratio of the quaternized ester-ammonium softening compound (a) to the substituted imidazoline ester softening compound (b) is preferably in the range of from about 0.04:1 to about 1.5:1, more preferably from about 0.25:1 to about 0.7:1.

Quaternized Ester-Ammonium Softening Comnound

The present invention contains as an essential component from about 1% to about 20%, preferably from about 1% to about 4% for dilute fabric softening compositions and from about 1% to about 5% for concentrated fabric compositions, of a quaternized ester-ammonium softening compound selected from the group consisting of:

(i)

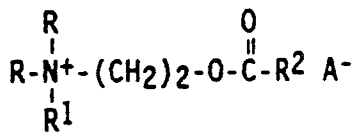

(ii)

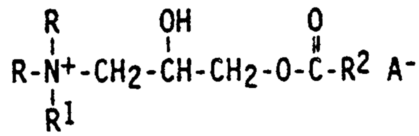

and mixtures thereof, wherein each R substituent is a short chain ($C_1$-$C_6$, preferably $C_1$-$C_3$) alkyl, alkenyl or hydroxyalkyl group, e.g., methyl (most preferred), ethyl, propyl, propenyl, hydroxyethyl, and the like, or mixtures thereof; $R^1$ is a long chain $C_{14}$-$C_{22}$ hydrocarbyl group, preferably $C_{16}$-$C_{18}$ alkyl, most preferably straight-chain $C_{18}$ alkyl; $R^2$ is a long chain $C_{13}$-$C_{21}$ hydrocarbyl group, preferably $C_{13}$-$C_{17}$ alkyl, most preferably $C_{17}$ straight chain alkyl. The counterion $A^-$ is not critical herein, and can be any softener-compatible anion, for example, chloride, bromide, methylsulfate, formate, sulfate, nitrate and the like. It will be understood that substituents R, $R^1$ and $R^2$ may optionally be substituted with various groups such as alkoxyl, hydroxyl, or can be branched. In addition R, $R^1$, and/or $R^2$ may optionally be unsaturated (i.e., alkenyl groups.) The preferred compounds can be considered to be mono-ester variations of ditallow dimethyl ammonium salts (e.g., DTDMAC, a widely used fabric softening compound).

The above compounds used as one of the active softener ingredients in the practice of this invention are prepared using standard reaction chemistry. For example, in a typical synthesis of a quaternary mono-ester composition softening compound of formula (i) above, an amine of the formula $RR^1NCH_2CH_2OH$ is esterified at the hydroxyl group with an acid chloride of the formula $R^2C(O)Cl$, then quaternized with an alkyl halide, RX, to yield the desired reaction product (wherein R, $R^1$ and $R^2$ are as defined in the present application). A method for the synthesis of a preferred mono-ester softening compound is disclosed in detail hereinafter. However, it will be appreciated by those skilled in the chemical arts that this reaction sequence allows a broad selection of compounds to be prepared. As illustrative, nonlimiting examples there can be mentioned the following quaternized mono-ester amines (wherein all long-chain alkyl substituents are straight-chain):

$[CH_3]_2[C_{18}H_{37}]^+NCH_2CH_2OC(O)C_{15}H_{31}Br^-$
$[CH_3]_2[C_{18}H_{37}]^+NCH_2CH_2OC(O)C_{17}H_{35}Br^-$
$[CH_3]_2[C_{16}H_{33}]^+NCH_2CH_2OC(O)C_{17}H_{35}Cl^-$
$[C_2H_5]_2[C_{17}H_{35}]^+NCH_2CH_2OC(O)C_{13}H_{27}Cl^-$
$[C_2H_5][CH_3][C_{18}H_{37}]^+NCH_2CH_2OC(O)C_{14}H_{29}CH_3SO_4^-$
$[C_3H_7][C_2H_5][C_{16}H_{33}]^+NCH_2CH_2OC(O)Cl_{15}H_{31}Cl^-$
$[iso\text{-}C_3H_7][CH_3][C_{18}H_{37}]^+NCH_2CH_2OC(O)Cl_{15}H_{31}I^-$

Similarly, in a typical synthesis of a quaternary mono-ester ammonium softening compound of formula (ii) above, a fatty acid of the formula $R^2COOH$ is neutralized with a tertiary amine of the formula $R^1N(R)_2$ and made into a fatty acid-tertiary ammonium salt which is then reacted with epichlorohydrin to yield the desired reaction product (wherein R, $R^1$ and $R^2$ are as defined above). A method for the synthesis of a preferred softening compound is disclosed in detail hereinafter. However, it will be appreciated by those skilled in the chemical arts that this reaction sequence allows a broad selection of compounds to be prepared.

Illustrative, nonlimiting examples of useful quaternized 2-hydroxypropyl monoester ammonium salts (wherein all long-chain alkyl substituents are straight-chain) include:

$[CH_3]_2[C_{18}H_{37}]^+NCH_2CH(OH)CH_2OC(O)C_{17}H_{35}Br^-$

$[CH_3]_2[C_{16}H_{33}]^+NCH_2CH(OH)CH_2OC(O)C_{15}H_{31}Cl^-$

$[C_2H_5]_2[C_{17}H_{35}]^+NCH_2CH(OH)CH_2OC(O)C_{15}H_{31}Cl^-$

$[C_2H_5][CH_3][C_{18}H_{37}]^+NCH_2CH(OH)CH_2OC(O)C_{17}H_{35}CH_3SO_4^-$

$[C_3H_7][C_2H_5][C_{16}H_{33}]^+NCH_2CH(OH)CH_2OC(O)C_{15}H_{31}Cl^-$

$[iso\text{-}C_3H_7][CH_3][C_{18}H_{37}]^+NCH_2CH(OH)CH_2OC(O)C_{15}H_{31}I^-$

Since the foregoing compounds are somewhat labile to hydrolysis, they should be handled rather carefully when used to formulate the compositions herein. For example, stable liquid compositions herein are preferably formulated at a pH in the range of about 2.0 to about 5.0, most preferably at a pH range of about 2.0 to about 3.5. The pH can be adjusted by the addition of a Bronsted acid. Examples of suitable Bronsted acids include the inorganic mineral acids, carboxylic acids, in particular the low molecular weight ($C_1$-$C_5$) carboxylic acids, and alkylsulfonic acids. Suitable inorganic acids include HCl, $H_2SO_4$, $HNO_3$ and $H_3PO_4$. Suitable organic acids include formic, acetic, methylsulfonic and ethylsulfonic acid. Preferred acids are hydrochloric and phosphoric acids.

Synthesis of a quaternized mono-ester amine softening compound of group (i)

Synthesis of the preferred biodegradable, quaternized mono-ester amine softening compound of group (i) can be accomplished using the following two-step process:

Step A. Synthesis of Amine

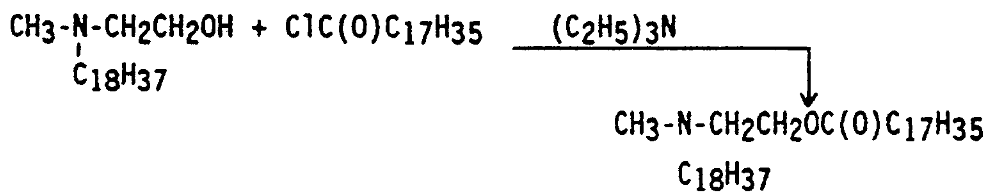


0.6 mole of octadecyl ethanol methyl amine is placed in a 3-liter, 3-necked flask equipped with a reflux condenser, argon (or nitrogen) inlet and two addition funnels. In one addition funnel is placed 0.4 moles of triethylamine and in the second addition funnel is placed 0.6 mole of palmitoyl chloride in a1:1 solution with methylene chloride. Methylene chloride (750 mL) is added to the reaction flask containing the amine and heated to 35° C water bath). The triethylamine is added dropwise, and the temperature is raised to 40-45° C while stirring over one-half hour. The palmitoyl chloride/methylene chloride solution is added dropwise and allowed to heat at 40-45° C under inert atmosphere overnight (12-16 h).

The reaction mixture is cooled to room temperature and diluted with chloroform (1500 mL). The chloroform solution of product is placed in a separatory funnel (4 L) and washed with sat. NaCl, dil. $Ca(OH)_2$, 50% $K_2CO_3$ (3 times)*, and, finally, sat. NaCl. The organic layer is collected and dried over $MgSO_4$, filtered and solvents are removed via rotary evaporation. Final drying is done under high vacuum (0.25 mm Hg).

(*Note: 50% $K_2CO_3$ layer will be below chloroform layer.)

ANALYSIS

TLC (thin layer chromatography)**: solvent system (75% diethyl ether: 25% hexane) Rf = 0.7.

IR ($CCl_4$): δ2910, 2850, 2810, 2760, 1722, 1450, 1370 $cm^{-1}$

$^1$H-NMR ($CDCl_3$): δ2.1-2.5 (8H), 2.1 (3H), 1.20 (58H), 0.9 (6H) ppm (relative to tetramethylsilane = 0 ppm).

(**10X20 cm pre-scored glass plates, 250 micron silica gel; visualization by PMA (phosphomolybdic acid - 5% in ethanol) staining.)

Step B: Quaternization

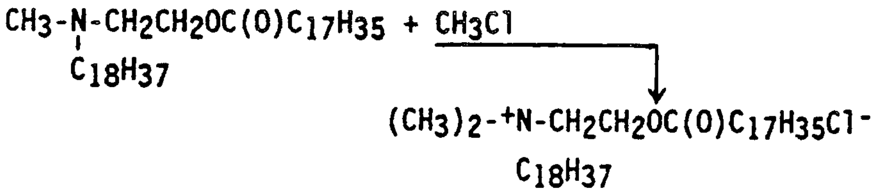

0.5 mole of the octadecyl palmitoyloxyethyl methyl amine, prepared in Step A, is placed in an autoclave sleeve along with 200-300 mL of acetonitrile (anhydrous). The sample is then inserted into the autoclave and purged three times with He (16275 mm Hg/21.4 ATM.) and once with $CH_3Cl$. The reaction is heated to 80° C under a pressure of 3604 mm Hg/4.7 ATM. $CH_3Cl$ and solvent are drained from the reaction mixture. The sample is dissolved in chloroform and solvent is removed by rotary evaporation, followed by drying on high vacuum (0.25 mm Hg). Both the $C_{18}H_{37}$ and $C_{17}H_{35}$ substituents in this highly preferred compound are n-alkyl.

<u>ANALYSIS</u>

TLC (5:1 chloroform:methanol)*: Rf = 0.25
IR ($CCl_4$): δ 2910, 2832, 1730, 1450 $cm^{-1}$.
$^1H$-NMR ($CDCl_3$): δ 4.0-4.5 (2H), 3.5 (6H), 2.0-2.7 (6H), 1.2-1.5 (58H), 0.9 (6H) ppm (relative to tetramethylsilane = 0 ppm).
$^{13}C$-NMR ($CDCl_3$): δ 172.5, 65.3, 62.1, 57.4, 51.8, 33.9, 31.8, 29.5, 28.7, 26.2, 22.8, 22.5, 14.0 (relative to tetramethylsilane = 0 ppm).
(*10X20 cm pre-scored glass plates, 250 micron silica gel; visualization by PMA staining.)

<u>Synthesis of quaternized mono-ester amine softening compound of group (ii)</u>

Synthesis of the preferred biodegradable, quaternized monoester ammonium softening compound of group (ii) can be accomplished using the following "one-pot" process:

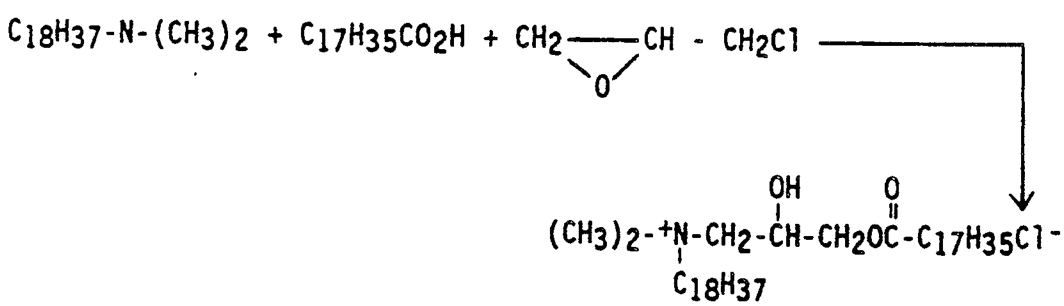

50 g (0.175 mole) of stearic acid and 80 g of isopropanol are placed in a 3-necked, 500-ml round bottom flask equipped with a reflux condenser, and mixed with a magnetic stir bar. 52.7g (0.175 mole) of dimethyl stearyl amine is added to the stirring stearic acid-isopropanol mixture, followed by the addition of 30 g of water. The mixture is stirred for approximately 1/2 hour (until a clear solution is obtained). Next, 18.4 g (0.190 mole) of epichlorohydrin is added to the mixture. The temperature is raised to 80° C (oil bath) and the stirring is continued for an additional 20 hours.

The reaction mixture is cooled to room temperature, and a white precipitate is formed. The solid material is collected on a Whatman® #1 filter paper (using a Buchi funnel apparatus) and is subsequently recrystallized from hot (60° C) isopropanol. The solid material is dried under vacuum (0.2 mm Hg) for 24 hours.

<u>ANALYSIS</u>

TLC (thin layer chromatography): 10X20 cm glass plate, 250 micron silica gel; solvent system, chloroform:methanol:water (15:6:0.6; v/v/v); visualization, 5% phosphomolybdic acid in ethanol; Rf = 0.61.
IR ($CCl_4$): δ3140, 2920, 2850, 1958, 1724, 1525, 1464, 1210, 1170 $cm^{-1}$.
$^1H$-NMR ($CDCl_3$): δ 6.02 (1H), 4.6-4.4 (1H), 4.3-40 (2H), 3.7-3.3 (10H), 2.3 (2H), 1.8-1.4 (4H), 1.2 (58H), 0.8 (6H) ppm (relative to tetramethylsilane).
$^{13}C$-NMR ($CDCl_3$): δ 173.4, 66.4, 66.0, 65.9, 63.5, 52.5, 52.4, 34.3, 31.9, 29.7, 29.5, 29.3, 29.2, 26.6, 25.0, 22.7, 22.6, 14.1 ppm (relative to tetramethylsilane).

Optionally, other types of quaternized ester-ammonium softening compounds, such as di-esters, can be used herein. Examples of such compounds include:

(i) 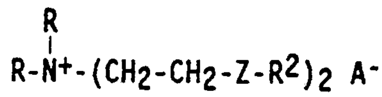

(ii) 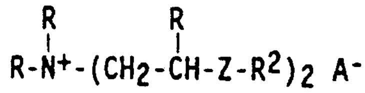

(iii) 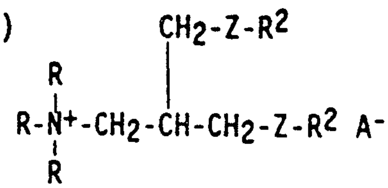

wherein

Z is 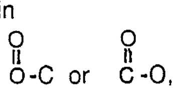, and R, $R^2$, and $A^-$ are as previously defined. The above list is intended to be illustrative of other types of ester-ammonium softening compounds which may optionally be used in the present invention and should not be construed as being limiting or inclusive.

Substituted Imidazoline Ester Softening Compound

The present invention contains as an essential component from about 1% to about 35%, preferably from about 4% to about 8% for dilute fabric softening compositions and from about 15% to about 25% for concentrated fabric softening compositions, of a substituted imidazoline ester softening compound having the formula

(b) 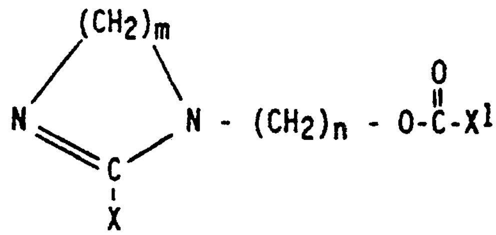

wherein X and $X^1$ are, independently, $C_{11}$-$C_{21}$ hydrocarbyl groups, preferably $C_{13}$-$C_{17}$ alkyl, most preferably straight chain $C_{17}$ alkyl, and m and n are, independently, from about 2 to about 4, preferably m and n are both 2. The total concentration of softener active cannot exceed about 40% by weight. It will be understood that substituents X and $X^1$ may optionally be substituted with various groups such as alkoxyl, hydroxyl, or can be branched, but such materials are not preferred herein. In addition X and/or $X^1$ may

optionally be unsaturated (i.e., alkenyl groups). The preferred substituted imidazoline ester softening compounds will hereinafter be referred to as di-alkyl imidazoline ester compounds.

The above compounds used as one of the active softener ingredients in the practice of this invention are prepared using standard reaction chemistry. For example, in a typical synthesis of a substituted imidazoline ester softening compound of formula (b) above, a fatty acid of the formula XCOOH is reacted with a hydroxyalkylenediamine of the formula $NH_2-(CH_2)_m-NH-(CH_2)_nOH$ to form an intermediate imidazoline precursor, which is then reacted with a methyl ester of a fatty acid of the formula:
$X^1COOCH_3$
to yield the desired reaction product (wherein X, $X^1$, m and n are as defined in the present application). A method for the synthesis of a preferred di-alkyl imidazoline ester softening compound is disclosed in detail hereinafter. However, it will be appreciated by those skilled in the chemical arts that this reaction sequence allows a broad selection of compounds to be prepared. As illustrative, nonlimiting examples there can be mentioned the following di-alkyl imidazoline esters (wherein all long-chain alkyl substituents are straight-chain)): 1-stearyl oxyethyl-2-stearyl imidazoline, 1-stearyl oxyethyl-2-palmityl imidazoline, 1-stearyl oxyethyl-2-myristyl imidazoline, 1-palmityl oxyethyl-2-palmityl imidazoline, 1-palmityl oxyethyl-2-myristyl imidazoline, 1-stearyl oxyethyl-2-tallow imidazoline, 1-myristyl oxyethyl-2-tallow imidazoline, 1-palmityl oxyethyl-2-tallow imidazoline, 1-coconut oxyethyl-2-coconut imidazoline, 1-tallow oxyethyl-2-tallow imidazoline, and mixtures of such imidazoline compounds.

Synthesis of a di-alkyl imidazoline ester softening compound

Synthesis of the preferred biodegradable di-alkyl imidazoline ester softening compound for use herein can be accomplished using the following process:

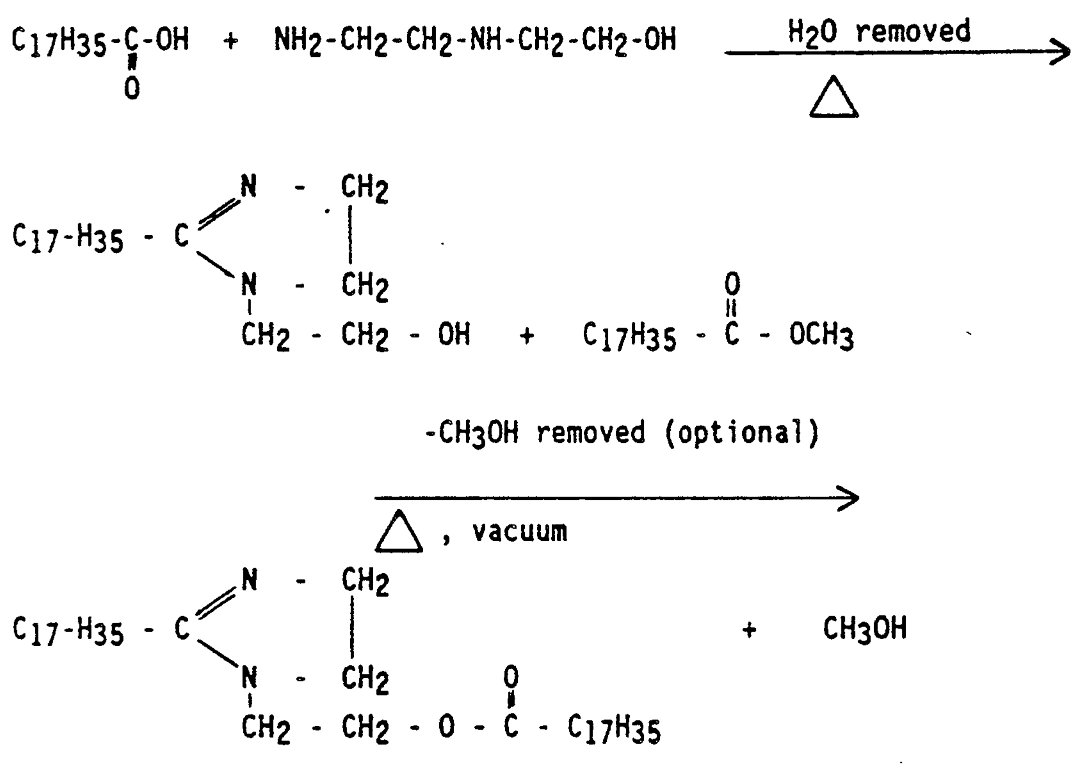


208.3g (2.0 moles) of β-hydroxyethylethylenediamine (aminoethylamino ethanol) are placed in a 3-necked 2 liter flask along with 426.8 g (1.5 moles) of stearic acid. The flask is sparged with argon and equipped with a reflux condenser, distillation apparatus and overhead stirrer. The reaction mixture is then heated to 165° C for 18 hours. Next, a vacuum of approximately 0.2 mm Hg is drawn for 4 hours, at a temperature of 165° C, during which time water and excess amine are collected. The reaction mixture is cooled to 120° C and 447.8 g (1.5 moles) of methyl stearate is added. Reaction temperature is increased to 170° C at which time the reaction flask is subjected to a vacuum of approximately 0.2 mm Hg for 22 hours. The above reaction

produces 1-stearyloxyethyl-2-stearyl imidazoline.

ANALYSIS

TLC (90:10:1.5 $CHCl_3$/MeOH/$NH_4OH$)*: Rf = 0.4.
IR ($CCl_4$): δ2920, 2850, 1740, 1615, 1460/1455, 1160 $CM^{-1}$
$^1$H-NMR ($CDCl_3$): δ4.1-4.3(2H), 3.2-3.7(6H), 2.1-2.5(4H), 1.3(60H), 0.9-1.1 (6H) ppm (relative to tetramethylsilane = 0 ppm).
$^{13}$C-NMR($CDCl_3$): δ173.3, 61.6, 52.2, 50.1, 45.5, 34.0, 31.8, 29.5, 27.6, 26.3, 24.7, 22.5, 13.9 ppm (relative to tetramethylsilane)

(*10X20 CM pre-scored glass plates, 250 microns.

Visualization by PMA (phosphomolybdic acid - 5% in ethanol) staining)

Optionally, other types of substituted imidazoline softening compounds can be used herein. Examples of such compounds include:

(i)

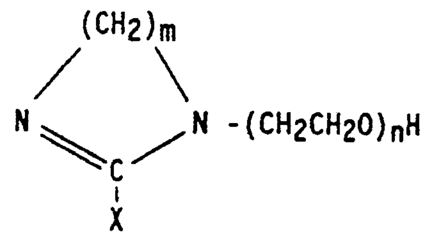

(ii)

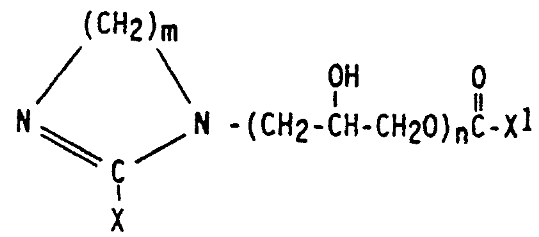

(iii)

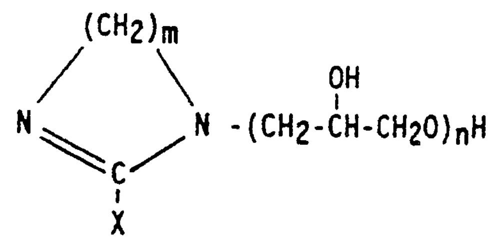

wherein X, X$^1$, m and n are as previously defined. The above list is intended to be illustrative of other types of substituted imidazoline softening compounds which may optionally be used in the present invention and should not be construed as being limiting or inclusive.

Liquid Carrier

The compositions herein comprise a liquid carrier, e.g., water, preferably a mixture of water and a $C_1$-$C_4$ monohydric alcohol (e.g., ethanol, propanol, isopropanol, butanol, and mixtures thereof), isopropanol being preferred. These compositions comprise from about 60% to about 98%, preferably from about 70% to about 95% of the liquid carrier. Preferably, the amount of the $C_1$-$C_4$ monohydric alcohol in the liquid carrier is from about 5% to about 50% by weight of the softening compounds, the balance of the liquid carrier being water.

The softening compounds used in this invention are insoluble in such water-based carriers and, thus, are present as a dispersion of fine particles therein. These particles are sub-micron, preferably having average diameters of from about 0.1 to about 0.5 microns in size and are conveniently prepared by high-shear mixing which disperses the compounds as fine particles. A method of preparation of a preferred dispersion is disclosed in detail in Examples I-III hereinafter. Again, since the softening compounds are hydrolytically labile, care should be taken to avoid the presence of base, and to keep the processing temperatures and pH within the ranges specified herein.

The particulate dispersions of the foregoing type can optionally be stabilized against settling by means of standard non-base emulsifiers, especially nonionic extenders. Such nonionics and their usage levels, have been disclosed in U.S. Patent 4,454,049, MacGilp et al., issued June 12, 1984, the disclosure of which is incorporated herein by reference.

Specific examples of nonionic extenders suitable for use in the compositions herein include glycerol esters (preferably glycerol monostearate), fatty alcohols (e.g., stearyl alcohol), ethoxylated linear alcohols (preferably Neodol 23-3 - the condensation product of a $C_{12}$-$C_{13}$ linear alcohol with 3 moles ethylene oxide, marketed by Shell Chemical Company) and mixtures thereof. Mixtures of glycerol monostearate and Neodol 23-3 are particularly preferred. The nonionic, if used, is typically used at a level of from about 0.1% to about 10% by weight of the composition.

## Optional Ingredients

Fully-formulated fabric softening compositions may optionally contain, in addition to the biodegradable quaternized ester-ammonium softening compounds and substituted imidazoline ester softening compounds of the formulas herein, and liquid carrier, one or more of the following ingredients.

## Conventional quaternary ammonium softening agents

The compositions of the present invention can further comprise a conventional di(higher alkyl) quaternary ammonium softening agent. The compositions herein can contain from 0% to about 25% (preferably from about 0.1% to about 10%) of the conventional di(higher alkyl) quaternary ammonium softening agent.

"Higher alkyl", as used in the context of the conventional quaternary ammonium salts herein, means alkyl groups having from about 8 to about 30 carbon atoms, preferably from about 11 to about 22 carbon atoms. Examples of such conventional quaternary ammonium salts include:

(i) acyclic quaternary ammonium salts having the formula:

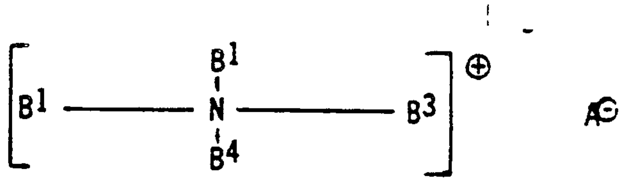

wherein $B^1$ is an acyclic aliphatic $C_{15}$-$C_{22}$ hydrocarbon group, $B^3$ is a $C_1$-$C_4$ saturated alkyl or hydroxyalkyl group, $B^4$ is selected from $B^1$ and $B^3$, and A is an anion;

(ii) diamido quaternary ammonium salts having the formula:

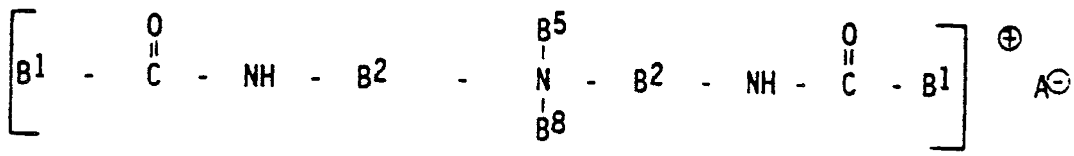

wherein $B^1$ is an acyclic aliphatic $C_{15}$-$C_{22}$ hydrocarbon group, $B^2$ is a divalent alkylene group having 1 to 3 carbon atoms, $B^5$ and $B^8$ are $C_1$-$C_4$ saturated alkyl or hydroxyalkyl groups, and A is an anion;

(iii)diamido alkoxylated quaternary ammonium salts having the formula:

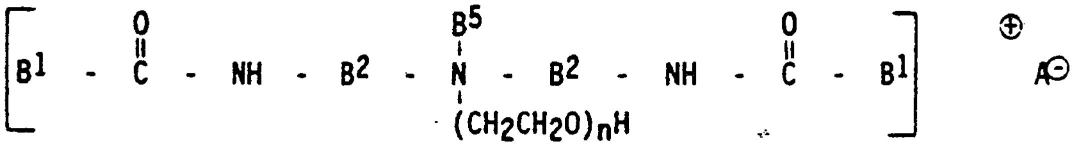

wherein n is equal to from about 1 to about 5, and $B^1$, $B^2$, $B^5$ and A are as defined above;

(iv) quaternary imidazolinium compounds having the formula:

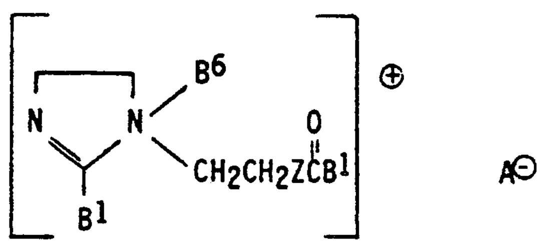

wherein $B^6$ is a $C_1$-$C_4$ saturated alkyl or H, Z is NH or O, and $B^1$ and A are as defined above.

Examples of Component (i) are the well-known dialkyldimethylammonium salts such as ditallowdimethylammonium chloride, ditallowdimethylammonium methylsulfate, di(hydrogenated tallow) dimethylammonium chloride, dibehenyldimethylammonium chloride.

Examples of Components (ii) and (iii) are methylbis(talloamidoethyl) (2-hydroxyethyl) ammonium methylsulfate and methylbis(hydrogenated tallowamidoethyl) (2-hydroxyethyl) ammonium methyl sulfate, wherein $B^1$ is an acyclic aliphatic $C_{15}$-$C_{17}$ hyrdocarbon group, $B^2$ is an ethylene group, $B^5$ is a methyl group . $B^8$ is a hydroxyalkyl group and A is a methylsulfate anion; these materials are available from Sherex Chemical Company under the trade names Varisoft® 222 and Varisoft® 110, respectively.

Examples of Component (iv) are 1-methyl-1-tallowamino-ethyl-2-tallowimidazolinium methylsulfate and 1-methyl-1-(hydrogenated tallowamidoethyl)-methylsulfate.

## Free amines

The liquid compositions herein should be substantially free (generally) less than about 1%) of free (i.e., unprotonated) amines.

Minor amounts of protonated amines, typically from about 0.05% to about 1.0%, namely primary, secondary and tertiary amines having, at least, one straight-chain organic group of from about 12 to about 22 carbon atoms may be used in the compositions of the present invention to enhance dispersion stability. Preferred amines of this class are ethoxyamines, such as monotallow-dipolyethoxyamine, having a total of from about 2 to about 30 ethoxy groups per molecule. Also suitable are diamines such as tallow-N,N′,N′-tris (2-hydroxyethyl)-1,3-propylenediamine, or $C_{16}$-$C_{18}$-alkyl-N-bis(2-hydroxyethyl)amines. Examples of the above compounds are those marketed under the trade names GENAMIN C, S, O and T, by Hoechst.

Care must be taken that if minor amounts of these amines are used to enhance the dispersion stability of the compositions, they are protonated with acid during formulation, otherwise the free amines may catalyze decomposition of the biodegradable fabric softening compounds during storage.

## Silicone Component

The fabric softening compositions herein optionally contain an aqueous emulsion of a predominantly linear polydialkyl or alkyl aryl siloxane in which the alkyl groups can have from one to five carbon atoms and may be wholly or partially fluoridated. These siloxanes act to provide improved fabric feel benefits. Suitable silicones are polydimethyl siloxanes having a viscosity, at 25 °C, of from about 100 to about 100,000 centistokes, preferably from about 1,000 to about 12,000 centistokes.

It has been found that the ionic charge characteristics of the silicone as used in the present invention

are important in determining both the extent of deposition and the evenness of distribution of the silicone and hence the properties of a fabric treated therewith.

Silicones having cationic character show an enhanced tendency to deposit. Silicones found to be of value in providing fabric feel benefits have a predominantly linear character and are preferably polydialkyl siloxanes in which the alkyl group is most commonly methyl. Such silicone polymers are frequently manufactured commercially by emulsion polymerization using a strong acid or strong alkali catalyst in the presence of a nonionic or mixed nonionic anionic emulsifier system. In addition to providing improved fabric feel benefits, the silicone components also improve the water absorbency of the fabrics treated with the softening compositions herein.

The optional silicone component embraces a silicone of cationic character which is defined as being one of:

(a) a predominantly linear di-$C_1$-$C_5$ alkyl or $C_1$-$C_5$ alkyl aryl siloxane, prepared by emulsion polymerization using a cationic or nonionic surfactant as emulsifier;

(b) an alpha-omega-di-quaternized di-$C_1$-$C_5$ alkyl or $C_1$-$C_5$ alkyl aryl siloxane polymer; or

(c) an amino-functional di-$C_1$-$C_5$ alkyl or alkyl aryl siloxane polymer in which the amino group may be substituted and may be quaternized and in which the degree of substitution (d.s.) lies in the range of from about 0.0001 to about 0.1, preferably from about 0.01 to about 0.075

provided that the viscosity at 25°C of the silicone is from about 100 to about 100,000 centistokes.

The fabric softening compositions herein may contain up to about 15%, preferably from about 0.1% to about 10%, of the silicone component.

Thickening Agent

Optionally, the compositions herein contain from 0% to about 3%, preferably from about 0.01% to about 2%, of a thickening agent. Examples of suitable thickening agents include: cellulose derivatives, synthetic high molecular weight polymers (e.g., carboxyvinyl polymer and polyvinyl alcohol), and cationic guar gums.

The cellulosic derivatives that are functional as thickening agents herein may be characterized as certain hydroxyethers of cellulose, such as Methocel®, marketed by Dow Chemicals, Inc.; also, certain cationic cellulose ether derivatives, such as Polymer JR-125®, JR-400®, and JR-30M®, marketed by Union Carbide.

Other effective thickening agents are cationic guar gums, such as Jaguar Plus ®, marketed by Stein Hall, and Gendrive 458 ®, marketed by General Mills.

Preferred thickening agents herein are selected from the group consisting of methyl cellulose, hydroxypropyl methylcellulose, hydroxybutyl methylcellulose, or mixtures thereof, said cellulosic polymer having a viscosity in 2% aqueous solution at 20°C of from about 15 to about 75,000 centipoise.

Soil Release Agent

Optionally, the compositions herein contain from 0% to about 10%, preferably from about 0.2% to about 5%, of a soil release agent. Preferably, such a soil release agent is a polymer. Polymeric soil release agents useful in the present invention include copolymeric blocks of terephthalate and polyethylene oxide or polypropylene oxide, and the like.

A preferred soil release agent is a copolymer having blocks of terephthalate and polyethylene oxide. More specifically, these polymers are comprised of repeating units of ethylene terephthalate and polyethylene oxide terephthalate at a molar ratio of ethylene terephthalate units to polyethylene oxide terephthalate units of from about 25:75 to about 35:65, said polyethylene oxide terephthalate containing polyethylene oxide blocks having molecular weights of from about 300 to about 2000. The molecular weight of this polymeric soil release agent is in the range of from about 5,000 to about 55,000.

Another preferred polymeric soil release agent is a crystallizable polyester with repeat units of ethylene terephthalate units containing from about 10% to about 15% by weight of ethylene terephthalate units together with from about 10% to about 50% by weight of polyoxyethylene terephthalate units, derived from a polyoxyethylene glycol of average molecular weight of from about 300 to about 6,000, and the molar ratio of ethylene terephthalate units to polyoxyethylene terephthalate units in the crystallizable polymeric compound is between 2:1 and 6:1. Examples of this polymer include the commercially available materials Zelcon® 4780 (from Dupont) and Milease® T (from ICI).

Highly preferred soil release agents are polymers of the generic formula:

$$X\text{-}(OCH_2CH_2)_n(O\text{-}\overset{\overset{O}{\|}}{C}\text{-}D^1\text{-}\overset{\overset{O}{\|}}{C}\text{-}OD^2)_u(O\text{-}\overset{\overset{O}{\|}}{C}\text{-}D^1\text{-}\overset{\overset{O}{\|}}{C}\text{-}O)\ (CH_2CH_2O\text{-})_n\text{-}X$$

in which X can be any suitable capping group, with each X being selected from the group consisting of H and alkyl or acyl groups containing from about 1 to about 4 carbon atoms. n is selected for water solubility and generally is from about 6 to about 113, preferably from about 20 to about 50. u is critical to formulation in a liquid composition having a relatively high ionic strength. There should be very little material in which u is greater than 10. Furthermore, there should be at least 20%, preferably at least 40%, of material in which u ranges from about 3 to about 5.

The $D^1$ moieties are essentially 1,4-phenylene moieties. As used herein, the term "the $D^1$ moieties are essentially 1,4-phenylene moieties" refers to compounds where the $D^1$ moieties consist entirely of 1,4-phenylene moieties, or are partially substituted with other arylene or alkarylene moieties, alkylene moieties, alkenylene moieties, or mixtures thereof. Arylene and alkarylene moieties which can be partially substituted for 1,4-phenylene include 1,3-phenylene, 1,2-phenylene, 1,8-naphthylene, 1,4-naphthylene, 2,2-biphenylene, 4,4-biphenylene and mixtures thereof. Alkylene and alkenylene moieties which can be partially substituted include ethylene, 1,2-propylene, 1,4-butylene, 1,5-pentylene, 1,6-hexamethylene, 1,7-heptamethylene, 1,8-octamethylene, 1,4-cyclohexylene, and mixtures thereof.

For the $D^1$ moieties, the degree of partial substitution with moieties other than 1,4-phenylene should be such that the soil release properties of the compound are not adversely affected to any great extent. Generally, the degree of partial substitution which can be tolerated will depend upon the backbone length of the compound, i.e., longer backbones can have greater partial substitution for 1,4-phenylene moieties. Usually, compounds where the $D^1$ comprise from about 50% to about 100% 1,4-phenylene moieties (from 0 to about 50% moieties other than 1,4-phenylene) have adequate soil release activity. For example, polyesters made according to the present invention with a 40:60 mole ratio of isophthalic (1,3-phenylene) to terephthalic (1,4-phenylene) acid have adequate soil release activity. However, because most polyesters used in fiber making comprise ethylene terephthalate units, it is usually desirable to minimize the degree of partial substitution with moieties other than 1,4-phenylene for best soil release activity. Preferably, the $D^1$ moieties consist entirely of (i.e., comprise 100%) 1,4-phenylene moieties, i.e., each $D^1$ moiety is 1,4-phenylene.

For the $D^2$ moieties, suitable ethylene or substituted ethylene moieties include ethylene, 1,2-propylene, 1,2-butylene, 1,2-hexylene, 3-methoxy-1,2-propylene and mixtures thereof. Preferably, the $D^2$ moieties are essentially ethylene moieties, 1,2-propylene moieties or mixtures thereof. Inclusion of a greater percentage of ethylene moieties tends to improve the soil release activity of compounds. Surprisingly, inclusion of a greater percentage of 1,2-propylene moieties tends to improve the water solubility of the compounds.

Therefore, the use of 1,2-propylene moieties or a similar branched equivalent is desirable for incorporation of any substantial part of the soil release component in the liquid fabric softener compositions. Preferably, from about 75% to about 100%, more preferably from about 90% to about 100%, of the $D^2$ moieties are 1,2-propylene moieties.

The value for each n is at least about 6, and preferably is at least about 10. The value for each n usually ranges from about 12 to about 113. Typically, the value for each n is in the range of from about 12 to about 43.

A more complete disclosure of these highly preferred soil release agents is contained in European Patent Application 185,427, Gosselink, published June 25, 1986, incorporated herein by reference.

## Viscosity Control Agents

Viscosity control agents can be used in the compositions of the present invention (preferably in concentrated compositions). Examples of organic viscosity modifiers are fatty acids and esters, fatty alcohols, and water-miscible solvents such as short chain alcohols. Examples of inorganic viscosity control agents are water-soluble ionizable salts. A wide variety of ionizable salts can be used. Examples of suitable salts include sodium citrate and the halides of the group IA and IIA metals of the Periodic Table of the Elements, e.g., calcium chloride, magnesium chloride, sodium chloride, potassium bromide and lithium chloride. Calcium chloride and sodium citrate are preferred. The ionizable salts are particularly useful during the process of mixing the ingredients to make the compositions herein, and later to obtain the desired viscosity. The amount of ionizable salts used depends on the amount of active ingredients used in the compositions and can be adjusted according to the desires of the formulator. Typical levels of salts used to control the composition viscosity are from about 20 to about 3,000 parts per million (ppm), preferably from

about 20 to about 2,000 ppm, by weight of the composition.

In addition to their role as viscosity agents, the ionizable salts mentioned above also function as electrolytes and can further improve the stability of the compositions herein. A highly preferred electrolyte is calcium chloride. Typical levels of use of the electrolyte are from about 20 to about 3,000 parts per million (ppm), preferably from about 20 to about 2,000 ppm by weight of the compositions.

Bactericides

Examples of bactericides used in the compositions of this invention include glutaraldehyde, formaldehyde, 2-bromo-2-nitro-propane-1,3-diol sold by Inolex Chemicals, located in Philadelphia, Pennsylvania, under the trade name Bronopol®, and a mixture of 5-chloro-2-methyl-4-isothiazoline-3-one and 2-methyl-4-isothiazoline-3-one sold by Rohm and Haas Company under the trade name Kathon® CG/ICP. Typical levels of bactericides used in the present compositions are from about 1 to about 1,000 ppm by weight of the composition.

Other Optional Ingredients

The present invention can include other optional components conventionally used in textile treatment compositions, for example, colorants, perfumes, preservatives, optical brighteners, opacifiers, fabric conditioning agents, surfactants, stabilizers such as guar gum and polyethylene glycol, anti-shrinkage agents, anti-wrinkle agents, fabric crisping agents, spotting agents, germicides, fungicides, anti-oxidants such as butylated hydroxy toluene, anti-corrosion agents, and the like.

In the method aspect of this invention, fabrics or fibers (including hair) are contacted with an effective amount, generally from about 20 ml to about 300 ml (per 3.5 kg of fiber or fabric being treated), of the compositions herein in an aqueous bath. Of course, the amount used is based upon the judgment of the user, depending on concentration of the composition, fiber or fabric type, degree of softness desired, and the like. Typically, about 120-250 ml. of a 8% dispersion of the softening compounds are used in a 25 l laundry rinse bath to soften and provide antistatic benefits to a 3.5 kg load of mixed fabrics. Preferable, the rinse bath contains from about 100 ppm to about 250 ppm of the fabric softening compositions herein.

Solid carrier materials can be used in place of liquids. For example, the softener compounds herein can be absorbed on particulate solids such as potassium sulfate, micronized silica, powdered urea, and the like, and added to a laundry rinse bath. Alternatively, the softeners can be releasably padded onto a sheet (e.g., paper toweling, nonwoven fabric, or the like) and tumbled with damp fabrics in a hot-air clothes dryer, in the manner of the BOUNCE® brand dryer-added product known in commercial practice. Generally, such solid-form compositions will comprise from about 1% to about 20% of the biodegradable fabric softening compounds, and from about 75% to about 99% of the solid carrier.

The following examples illustrate the practice of the present invention but are not intended to be limiting thereof.

EXAMPLE I

A storage stable biodegradable fabric softening composition of the present invention is made as follows:

| Ingredient | Percent (wt.) |
|---|---|
| 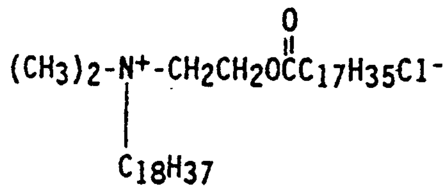 | 4.0% |
| 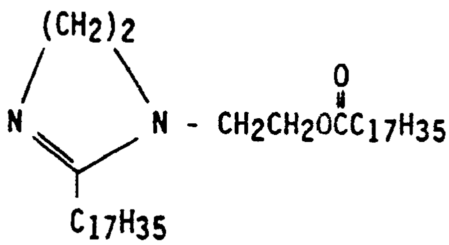 | 4.0% |
| Isopropanol | 2.0% |
| Bronopol® | 0.01% |
| Dye | 20 ppm |
| HCl | 0.30% |
| $CaCl_2$ | 0.02% |
| Water | Balance |

20 g of the biodegradable quaternized monoester-ammonium softening compound, 20 g of the biodegradable dialkyl imidazoline ester softening compound and 15 g of isopropanol are mixed and heated to 80 °C to form a fluidized "melt". The molten mixture is then poured into a 460 g water seat containing 1.5g of HCl with high shear mixing. The water is preheated to 70 °C, and 20 ppm blue dye and 100 ppm Bronopol® are added to the water prior to mixing. About 2 g of the isopropanol is evaporated from the molten mixture before it is poured into the water. The dispersion is mixed for 15 minutes at 7000 rpm (Tekmar high shear mixer). During mixing the temperature of the dispersion is maintained within 70-75 °C by a cooling water bath. 0.5 g of $CaCl_2$ (in a 5% solution) is added while mixing to reduce viscosity. The resulting dispersion will have a viscosity of about 100 centipoise (at 25 °C), a pH of about 3.7, and will contain less than about 1% of free (i.e., unprotonated) amines. The average particle size in the dispersion will typically be about 0.30 microns.

In an alternative preferred embodiment, prepared according to the procedure described above, the composition can comprise: 3.0 wt. percent biodegradable quaternized monoesterammonium softening compound; 5.0 wt. percent biodegradable dialkyl imidazoline ester softening compound; 2.0 wt. percent isopropanol; 0.01 wt. percent Bronopol®; 20 ppm dye; 0.30 wt. percent HCl; 0.02 wt. percent CaCl2; and a balance of water.

## EXAMPLE II

A storage stable biodegradable fabric softening composition of the present invention is made as follows:

| Ingredient | Percent (wt.) |
|---|---|
| 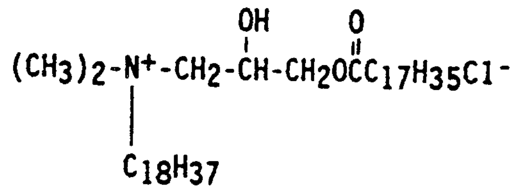 | 3.0% |
| 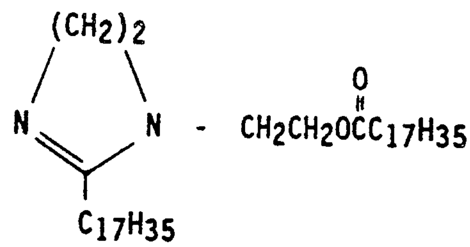 | 6.0% |
| Isopropanol | 1.5% |
| Polydimethylsiloxane (PDMS) | 1.0% |
| HCl | 0.5% |
| $CaCl_2$ | 0.03% |
| Water | Balance |

15 g of the biodegradable quaternized monoester-ammonium softener compound, 30 g of the biodegradable dialkyl imidazoline ester and 9.5 g of isopropanol are mixed and heated to 75° C to form a fluidized "melt". The molten mixture is then poured into a 450 g water seat with high shear mixing. The water is preheated to 70° C, and 2.5 g of HCl and 5 g of PDMS are added to the water prior to mixing. About 2 g of the isopropanol is evaporated from the molten mixture before it is poured into the water. The dispersion is mixed for 15 minutes at 7000 rpm (Tekmar high shear mixer). During mixing the temperature of the dispersion is maintained within 70-75° C by a cooling water bath. 0.15 g of $CaCl_2$ (in a 5% solution) is added while mixing to reduce viscosity. The resulting dispersion will have a viscosity of about 80 centipoise (at 25° C), a pH of about 3.9, and will contain less than about 1% of free (i.e., unprotonated) amines. The average particle size in the dispersion will typically be about 0.25 microns.

## EXAMPLE III

A storage stable biodegradable concentrated fabric softening composition of the present invention is made as follows:

| Ingredient | Percent (wt.) |
|---|---|
| 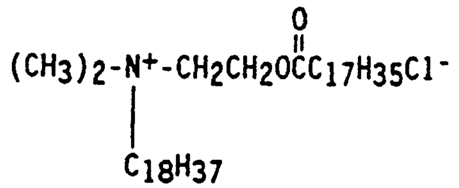 | 6.0% |
| 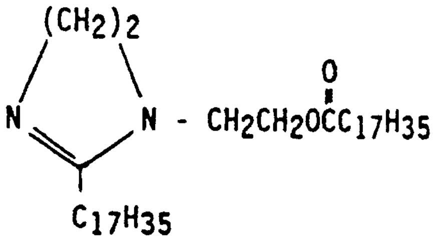 | 14.0% |
| Isopropanol | 3.0% |
| HCl | 1.0% |
| $CaCl_2$ | 0.1% |
| Water | Balance |

30 g of the biodegradable quaternized monoester-ammonium softener compound, 70 g of the biodegradable dialkyl imidazoline ester and 19 g of isopropanol are mixed and heated to 75° C to form a fluidized "melt". The molten mixture is then poured into a 400 g water seat with high shear mixing. The water is preheated to 70° C, and 5 g of HCl is added to the water prior to mixing. About 4 g of the isopropanol is evaporated from the molten mixture before it is poured into the water. The dispersion is mixed for 15 minutes at 7000 rpm (Tekmar high shear mixer). During mixing the temperature of the dispersion is maintained within 70-75° C by a cooling water bath. 0.5 g of $CaCl_2$ is added (in a 5% solution) while mixing to reduce viscosity. The resulting dispersion will have a viscosity of about 90 centipoise (at 25° C), a pH of about 2.8. and will contain less than about 1% of free (i.e., unprotonated) amines. The average particle size of the dispersion will typically be about 0.3 microns.

In a convenient mode, this concentrated composition is packaged in a simple plastic pouch, which is opened and poured into 4X its volume of water prior to use to prepare a "single strength" softener composition, thereby saving on packaging and shipping costs, as well as storage space.

## EXAMPLE IV

A storage stable biodegradable fabric softening composition of the present invention is made as follows:

| Ingredient | Percent (wt.) |
|---|---|
| 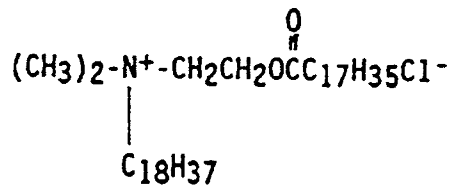 | 5.0% |
| 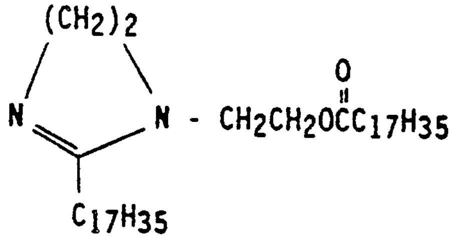 | 17.0% |
| Polydimethylsiloxane (PDMS) | 0.5% |
| Polyethylene gylcol | 1.2% |
| Dye | 60 ppm |
| HCl | 1.0% |
| $CaCl_2$ | 0.2% |
| Perfume | 0.75% |
| Water | Balance |

25 g of the biodegradable quaternized monoester-ammonium softening compound, 85 g of the biodegradable dialkyl imidazoline ester softening compound are mixed and heated to 80 °C to form a fluidized "melt". The molten mixture is then poured into a 360 g water seat containing 7.5 g of HCl with high shear mixing. The water is preheated to 70 °C, and 15 g of PDMS and 60 ppm blue dye are added to the water prior to mixing. The dispersion is mixed for 15 minutes at 7000 rpm (Tekmar high shear mixer). During mixing the temperature of the dispersion is maintained within 70-75 °C by a cooling water bath. 1 g of $CaCl_2$ (in a 5% solution) is added while mixing to reduce viscosity. After the dispersion has cooled to 38 °C, 6 g of polyethylene glycol (as a 50% solution) and 3.75 g perfume are added while mixing at 5000 rpm for 1 minute. The resulting dispersion will have a viscosity of about 100 centipoise (at 25 °C), a pH of about 2.8, and will contain less than about 1% of free (i.e., unprotonated) amines. The average particle size in the dispersion will typically be about 0.3 microns.

## EXAMPLE V

A storage stable biodegradable fabric softening composition of the present invention is made as follows:

| Ingredient | Percent (wt.) |
|---|---|
| 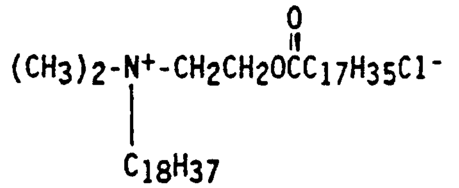 | 2.0% |
| 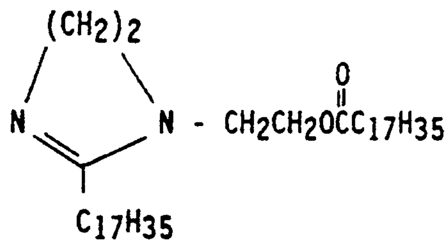 | 30.0% |
| Isopropanol | 1.5% |
| Bronopol® | 100 ppm |
| Dye | 120 ppm |
| HCl | 3.0% |
| Sodium Citrate | 2000 ppm |
| Perfume | 0.75% |
| Water | Balance |

10 g of the biodegradable quaternized monoester-ammonium softening compound, 150 g of the biodegradable dialkyl imidazoline ester softening compound and 10.5 g of isopropanol are mixed and heated to 80° C to form a fluidized "melt". The molten mixture is then poured into a 285 g water seat containing 15 g of HCl with high shear mixing. The water is preheated to 70° C, and 120 ppm blue dye and 100 ppm Bronopol® are added to the water prior to mixing. About 3 g of the isopropanol is evaporated from the molten mixture before it is poured into the water. The dispersion is mixed for 15 minutes at 7000 rpm (Tekmar high shear mixer). During mixing the temperature of the dispersion is maintained within 70-75° C by a cooling water bath. 1 g of sodium citrate (in a 5% solution) is added while mixing to reduce viscosity. After the dispersion is cooled to 38°C, 3.75 g perfume is added while mixing at 5,000 rpm for 1 minute. The resulting dispersion will have a viscosity of about 30 centipoise (at 25° C), a pH of about 2.8, and will contain less than about 1% of free (i.e., unprotonated) amines. The average particle size in the dispersion will typically be about 0.25 microns.

Typically, the liquid fabric softening compositions in the above examples are added to the rinse cycle of conventional washing machines. When multiple rinses are used, the fabric softening composition is preferably added to the final rinse. The amount added to the rinse cycle is generally from about 20 ml to about 300 ml (per 3.5 kg of fabric being treated) of the compositions of Examples I-II (and the diluted versions of Examples III-V).

Importantly, the above biodegradable liquid compositions (Examples I-V) will display excellent softening characteristics on both natural and synthetic fabrics, low viscosity at both normal and elevated temperatures, and good product stability, concentratability, and dispersibility.

## EXAMPLE VI

The preparation of a fabric-softener sheet for use in a hot-air clothes dryer is as follows:

| Fabric Conditioning Composition Components | Percent (wt.) |
|---|---|
| 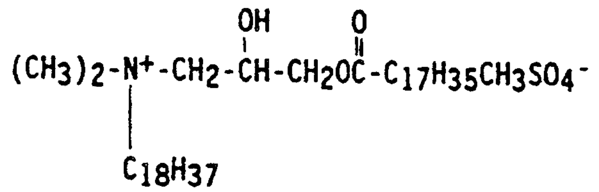 | 20.0% |
| 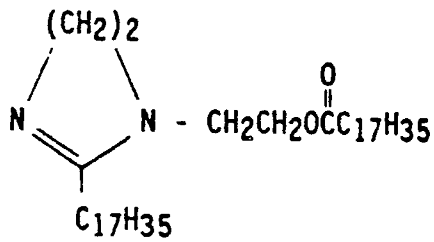 | 26.0% |
| Sorbitan Monostearate | 46.0% |
| Clay | 7.0% |
| Perfume | 1.0% |

| Dryer-added Sheet Substrate Composition | |
|---|---|
| Rayon fibers | 70.0% |
| Polyvinyl acetate | 30.0% |
| (10"x14" (25.4 cm x 35.6 cm) sheets, 1.4 g) | |

The biodegradable quaternized monoester-ammonium softener compound, biodegradable dialkyl imidazoline ester softener compound, sorbitan monostearate, clay (Bentolite L, a montmorillonite clay, obtainable from Southern Clay Products), and perfume are mixed and heated to 80 °C to form a fluidized "melt". The substrate (made of the rayon fibers with polyvinyl acetate) is then coated with about 4 grams of the molten actives and dried overnight. This provides a weight ratio of fabric conditioning composition:dry substrate of approximately 3.

Following solidification of the fabric conditioning composition, the substrate is slit with a knife, said slits being in substantially parallel relationship and extending to within about 1 inch (2.5 cm) from at least one edge of said substrate. The width of an individual slit is approximately 0.2 inches (0.5 cm). These dryer added sheets are added to a clothes dryer together with damp fabrics to be treated (typically, one sheet per 3.5 kg load of fabrics, dry weight basis). The heat and tumbling action of the revolving dryer drums evenly distributes the composition over all fabrics, and dries the fabrics. Fabric softening and static control are provided to the fabrics in this manner.

## Claims

1. A liquid fabric softening and antistatic composition comprising a quaternized ester-ammonium softening compound, a substituted imidazoline ester softening compound and a liquid carrier, characterized in that the composition contains:

(a) from 1% to 20% by weight of a quaternized ester-ammonium softening compound selected from the group consisting of:

(i) 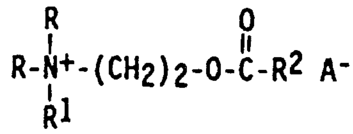

(ii) 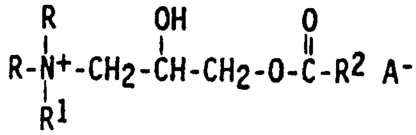

and mixtures thereof, wherein each R substituent is independently selected from $C_1$-$C_6$ alkyl, alkenyl or hydroxyalkyl groups, preferably $C_1$-$C_3$ alkyl, $R^1$ is a $C_{14}$-$C_{22}$ hydrocarbyl group, preferably $C_{16}$-$C_{18}$ alkyl, $R^2$ is a $C_{13}$-$C_{21}$ hydrocarbyl group, preferably $C_{13}$-$C_{17}$ alkyl, and $A^-$ is a softener compatible anion;

(b) from 1% to 35% by weight of a substituted imidazoline ester softening compound having the formula

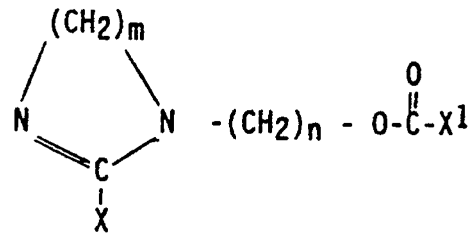

wherein X and X' are, independently, $C_{11}$-$C_{21}$ hydrocarbyl groups, preferably $C_{13}$-$C_{17}$ alkyl, and m and n are, independently, from 2 to 4, preferably 2; provided that the total concentration of component (a) and component (b) softening compounds does not exceed 40% by weight; and

(c) from 60% to 98% of a liquid carrier.

2. A composition according to Claim 1 wherein the liquid carrier consists of a mixture of
(a) $C_1$-$C_4$ monohydric alcohol, or mixtures thereof, preferably isoproponal; and
(b) water;
the amount of monohydric alcohol being from 5% to 50% by weight of the softening compounds.

3. A composition according to Claim 1 or Claim 2 wherein the softening compounds are present as particles, preferably having an average diameter in the range of from 0.1 to 0.5 micron, dispersed in the liquid carrier and which optionally further contains from 20 to 3,000 ppm of a salt selected from the group consisting of calcium chloride, magnesium chloride, sodium chloride, potassium chloride, lithium chloride, and mixtures thereof, preferably calcium chloride.

4. A composition according to any one of Claims 1 to 3 which is substantially free of unprotonated amines and which optionally contains from 0.1% to 10% by weight of a nonionic extender, preferably selected from the group consisting of glycerol esters, fatty alcohols, ethoxylated linear alcohols, and mixtures thereof.

5. A composition according to any one of Claims 1 to 4 which is formulated at a pH of from 2.0 to 5.0, preferably at a pH from 2.0 to 3.5.

6. A composition according to any one of Claims 1 to 5 wherein the ratio of the quaternized ester-ammonium softening compound (a) to the substituted imidazoline ester softening compound (b) is within the range of from 0.1:1 to 1.5:1, preferably from 0.25:1 to 0.7:1.

7. A composition according to any one of Claims 1 to 6 which contains from 2% to 4% of the quaternized ester-ammonium softening compound of the formula

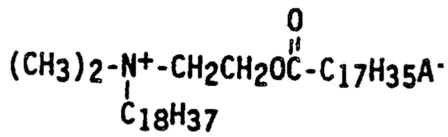

and from 4% to 6% of the substituted imidazoline ester softening compound of the formula

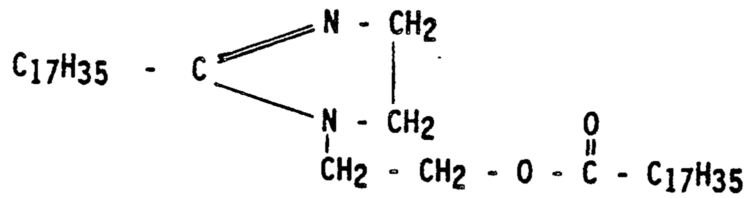

8. A composition according to any one of Claims 1 to 6 which contains from 3% to 5% by weight of the quaternized ester-ammonium softening compound of the formula

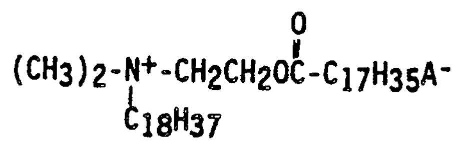

and from 15% to 20% of the substituted imidazoline ester softening compound of the formula

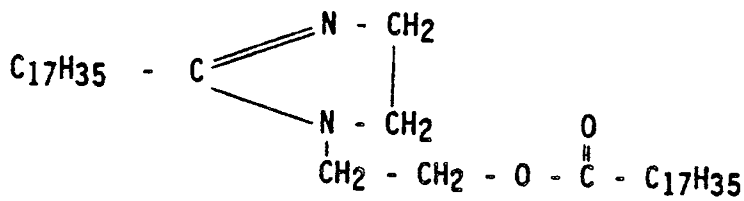

9. A fabric softening and antistatic composition in solid form comprising a quaternized ester-ammonium softening compound, a substituted imidazoline ester softening compound and a solid carrier, characterized in that the composition contains:

(a) from 1% to 40% by weight of a quaternized ester-ammonium softening compound selected from the group consisting of:

(i) 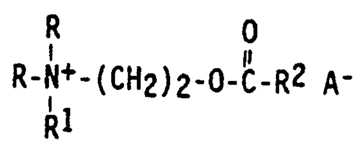

(ii) 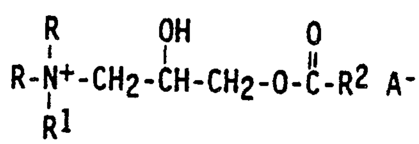

and mixtures thereof, wherein each R substitutent is independently selected from $C_1$-$C_6$ alkyl, alkenyl or hydroxyalkyl groups, $R^1$ is a $C_{14}$-$C_{22}$ hydrocarbyl group, $R^2$ is a $C_{13}$-$C_{21}$ hydrocarbyl group, and $A^-$ is a softener compatible anion;

(b) from 1% to 40% by weight of a substituted imidazoline ester softening compound having the formula

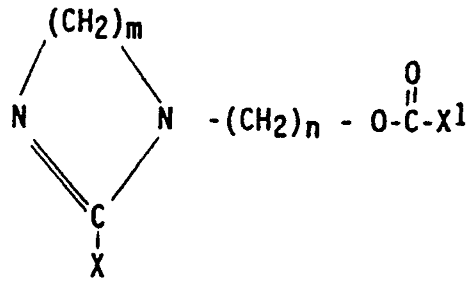

wherein X and X' are, independently, $C_{11}$-$C_{21}$ hydrocarbyl groups, and m and n are, independently, from 2 to 4; and

(c) a solid carrier, preferably a sheet substrate;

said softener compounds being releasably affixed to said solid carrier; wherein the ratio of the quaternzied ester-ammonium softening compound (a) to the substituted imidazoline ester softening compound (b) preferably is within the range of from 0.1:1 to 1.5:1.

10. A method of softening and providing an antistatic finish to fabrics by contacting said fabrics with an effective amount of a composition according to any one of Claims 1 to 9.